# EUROPEAN PATENT APPLICATION

(11) **EP 2 329 824 A1**
(43) Date of publication of application: **08.06.2011**
(21) Application number: 09813104.8
(22) Date of filing: 10.09.2009
(51) Int. Cl.: A61K 31/5375, A61P 25/04, C07D 265/30

(54) **NOVEL PHARMACEUTICAL COMPOSITION FOR TREATMENT OF NOCICEPTIVE PAIN**

(30) Priority: 11.09.2008 JP 2008234069
(71) Applicant: Astellas Pharma Inc., Tokyo 103-8411 (JP)
(72) Inventor: TAKESHITA, Nobuaki, Tokyo 103-8411 (JP); NISHIGAKI, Fusako, Tokyo 103-8411 (JP); AOKI, Toshiaki, Tokyo 103-8411 (JP); TAMURA, Seiji, Tokyo 103-8411 (JP); KIKUCHI, Kazumi, Tokyo 103-8411 (JP); KURODA, Akio, Tokyo 103-8411 (JP)
(74) Representative: Albrecht, Thomas
(86) International application number: PCT/JP2009/065791
(87) International publication number: WO 2010/029958

(57) **Abstract**

Provided is a novel potent pharmaceutical composition for treating nociceptive pain.

The present invention relates to a pharmaceutical composition for treating nociceptive pain, containing a morpholine derivative or a pharmaceutically acceptable salt thereof, as an active ingredient. The present invention is useful in providing an excellent pharmaceutical composition for treating nociceptive pain. In addition, the present invention is particularly useful in providing a pharmaceutical composition for treating pain accompanying a disease selected from the group consisting of rheumatoid arthritis, rheumatoid spondylitis, osteoarthritis, spondylosis deformans, gouty arthritis, juvenile arthritis, scapulohumeral periarthritis, and cervical syndrome, lumbago, lumbago accompanying spondylosis deformans, menalgia, pain and tumentia after inflammation, operation or injury, pain after odontectomy, and cancer pain. In addition, the present invention is particularly useful for alleviating pain in a damaged cartilage region, and is particularly useful for osteoarthritis in which NSAIDs are not effective.

## Description

### Technical Field

The present invention relates to a novel pharmaceutical composition for treating nociceptive pain, containing a morpholine derivative or a pharmaceutically acceptable salt thereof.

### Background Art

Pain is a self-conscious combined sensation associated with actual or potential tissue damage and emotional response there to, which come in many varieties. In addition, pain is broadly classified into somatogenic pain and psychogenic pain, and the former is classified into nociceptive pain and neuropathic pain. Nociceptive pain is caused by external stimuli or endogenous pathology. Nociceptive pain is divided into acute diseases and chronic diseases, but is mostly acute pain which disappears when the underlying disease is cured, which acts as a biological signal for disorders. Neuropathic pain is chronic pain caused by dysfunction of nervous systems in peripheral or central nerves, which includes diabetes-derived pain, nerve compression, spinal injuries and the like. Psychogenic pain is organically unexplained chronic pain which is caused by a mental disorder rather than a physical disorder and includes chronic headaches, unknown stomach aches and the like. In such a pain, chronic (constant) pain is a target to be treated due to the serious suffering of the patients. In particular, regarding chronic pain accompanying arthritis, diabetes, cancers and the like, there is a need for treatment of the underlying disease but also for treatment of the associated pain, but conventional analgesic agents have unsatisfactory efficacy and safety.

In nociceptive pain, tissue associated with joints such as bones or cartilage is involved in the onset of chronic pain accompanying arthritis. Cartilage tissue is a tissue composed of cartilage cells and cartilage substrates, which forms skeletal systems with bones. Osteoarthritis is a disease in which articular cartilage is chronically worn or lost, and the cartilage is deformed. Osteoarthritis includes two kinds of osteoarthritis (*i.e.*, primary and secondary osteoarthritis). Primary osteoarthritis is caused by factors such as muscular degeneration, obesity or mechanical stress and secondary osteoarthritis is caused by clear factors such as injury or diseases. Rheumatoid arthritis is a disease which is characterized by unexplained chronic arthritis and causes inflammation of articular synovium, if progressed, destruction of cartilage and bones or articular deformation.
In arthritis, osteoarthritis involves pain as a main symptom and may often result in the patient becoming bedridden due to chronically continuous pain as well as putting a large burden on the patient, although it does not entail serious inflammation. It was reported that 26% of patients with osteoarthritis in the knee or crotch joint suffer from considerably serious pain (J Rheumatol, 23, 1037, 1996). For such patients, therapeutic effects of non-steroidal anti-inflammatory agents or cyclooxygenase-2 inhibitors generally used for pain of osteoarthritis are limited. In addition, there is a need for creation of an agent for treating pain accompanying arthritis which is highly effective and exhibits reduced side-effects from the viewpoint that these drugs have the risk of gastrointestinal disorders and cardiovascular disorders when used for a long period of time.

In addition to steroidal agents and non-steroidal anti-inflammatory agents and cyclooxygenase-2 inhibitors, drugs which are known to have serotonin and norepinephrine reuptake inhibitory (SNRI) action, increase their monoamine concentrations in synaptic clefts and thus exhibit analgesic action via a descending pain pathway and drugs through opioid receptors are used for treating pain accompanying osteoarthritis.

Recently, it has been reported that (S)-N-methyl-y-(1-naphthalenyloxy)-2-thiophenepropanamine (duloxetine) which improved the side effect profiles of the tricyclic antidepressants shows an efficacy in patients who suffered from osteoarthritis (Non-patent Citation 1).

Meanwhile, (±)-2-[(inden-7-yloxy)methyl]morpholine hydrochloride (indeloxazine hydrochloride) is known to exhibit a high affinity to a serotonin and norepinephrine uptake site and has a serotonin and norepinephrine reuptake inhibitory activity in brains of rats and an antidepressive activity, and had been used for the treatment of psychiatric symptoms involving cerebrovascular disorders in Japan and South Korea (Patent Citation 1 and Non-patent Citation 2). In addition, its optically active substance, (+)-2-[(inden-7-yloxy)methyl]morpholine hydrochloride is also known to exhibit a serotonin and norepinephrine reuptake inhibitory activity in the same manner as (±)-2-[(inden-7-yloxy)methyl]morpholine hydrochloride (Non-patent Citation 3), and to be useful for a neuropathic pain and diabetic neuropathic disorder animal model (Patent Citations 2 and 3).

Duloxetine, amitriptyline and the like are known to be effective in a rat inflammatory pain model (Non-patent Citation 4 and Non-patent Citation 5). In addition, a serotonin uptake inhibitor was also reported to be effective in arthrosis (Patent Citation 4). However, it has not been reported that a morpholine derivative containing indeloxazine or its optically active substance, (+)-2-[(inden-7-yloxy)methyl]morpholine, is effective in nociceptive pain of rheumatoid arthritis or osteoarthritis.

### Related Art Document

### Patent Citation

Patent Citation 1: US Patent No. 4109088
Patent Citation 2: The pamphlet of International Publication WO 2008/111668
Patent Citation 3: The pamphlet of International Publication WO 2008/111669
Patent Citation 4: The pamphlet of International Publication WO 2008/090331 Non patent Citation

Non patent Citation 1: The European League Against Rheumatism (EULAR) Congress) (European Rheumatism Congress) 2008, FRI0338
Non patent Citation 2: Neuropharmacology, 1998, vol. 37, p1169-1176
Non patent Citation 3: Chemical and Pharmaceutical Bulletin, 1985, Vol. 33, No. 9, p3766-3774
Non patent Citation 4: The Journal of Pharmacology And Experimental Therapeutics, 2004, Vol. 311, p576-584
Non patent Citation 5: The Journal of Neuroscience, 2007, Vol. 27, p6045-6053

### Disclosure of Invention

### Technical Problem

It is an object of the present invention to provide a novel and excellent pharmaceutical composition for treating nociceptive pain.

### Problems to Be Solved by the Invention

The present inventors found that a morpholine derivative or a pharmaceutically acceptable salt thereof exhibits a potent analgesic action as a result of evaluation of nociceptive pain in monoiodoacetate (MIA)-induced osteoarthritis models in which osteoarthritis is onset, adjuvant-induced arthritis rat models used for evaluation of inflammatory pain or tumentia, and bradykinin (BK)-induced knee joint pain models, and thus accomplished the present invention.

It is an object of the present invention to provide a pharmaceutical composition for treating nociceptive pain, comprising a morpholine derivative or a pharmaceutically acceptable salt thereof as an active ingredient, that is, an agent for treating nociceptive pain, containing a morpholine derivative or a pharmaceutically acceptable salt thereof.

It is another object of the present invention to provide use of a morpholine derivative or a pharmaceutically acceptable salt thereof for the manufacture of a medicament for treating nociceptive pain.

It is a further object of the present invention to provide a method for treating nociceptive pain, comprising administering an effective amount of a morpholine derivative or a pharmaceutically acceptable salt thereof to human.

It is a further object of the present invention to provide a method for producing a pharmaceutical composition for treating nociceptive pain, comprising mixing a morpholine derivative or a pharmaceutically acceptable salt thereof, with a pharmaceutically acceptable carrier, vehicle or excipient.

It is a further object of the present invention to provide a commercial package comprising a pharmaceutical composition comprising the morpholine derivative or a pharmaceutically acceptable salt thereof as an active ingredient, and a description in which the morpholine derivative or a pharmaceutically acceptable salt thereof may be used or should be used for treating nociceptive pain.

The present invention relates to a pharmaceutical composition for treating nociceptive pain, comprising a morpholine derivative represented by the formula (I) or a pharmaceutically acceptable salt thereof as an active ingredient: 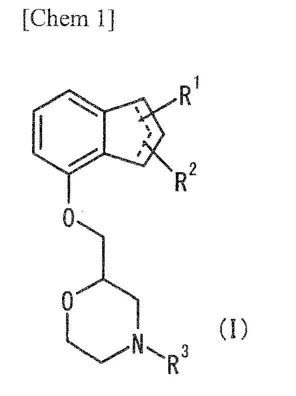
wherein R¹ and R² are the same or different and represent hydrogen, lower alkyl or phenyl;
R³ represents hydrogen, lower alkyl, phenyl or benzyl; and
a dotted line indicates that a double bond may be formed.

Further, the present invention relates to a pharmaceutical composition for treating nociceptive pain, comprising 2-[(inden-7-yloxy)methyl]morpholine or a pharmaceutically acceptable salt thereof as an active ingredient.

Further, the present invention relates to a pharmaceutical composition for treating nociceptive pain, comprising (±)-2-[(inden-7-yloxy)methyl]morpholine or a pharmaceutically acceptable salt thereof as an active ingredient.

Further, the present invention relates to a pharmaceutical composition for treating nociceptive pain, comprising (+)-2-[(inden-7-yloxy)methyl]morpholine or a pharmaceutically acceptable salt thereof as an active ingredient.

### Effects of the Invention

The present invention is useful in providing an excellent pharmaceutical composition for treating nociceptive pain. In addition, the present invention is particularly useful in providing a pharmaceutical composition for treating pain accompanying a disease selected from the group consisting of rheumatoid arthritis, rheumatoid spondylitis, osteoarthritis, spondylosis deformans, gouty arthritis, juvenile arthritis, scapulohumeral periarthritis, and cervical syndrome; lumbago; lumbago accompanying spondylosis deformans; menalgia; pain and tumentia after inflammation, operation or injury; pain after odontectomy; and cancer pain.
In addition, the present invention is particularly useful for alleviating pain in a damaged cartilage region, and is particularly useful for osteoarthritis in which NSAIDs are not effective.

### Best Mode for Carrying Out the Invention

Hereinafter, preferred embodiments of the present invention are described.
(1) A pharmaceutical composition for treating pain accompanying a disease selected from the group consisting of rheumatoid arthritis, rheumatoid spondylitis, osteoarthritis, spondylosis deformans, gouty arthritis, juvenile arthritis, scapulohumeral periarthritis, and cervical syndrome; lumbago; lumbago accompanying spondylosis deformans; menalgia; pain and tumentia after inflammation, operation or injury; pain after odontectomy; and cancer pain, containing a morpholine derivative represented by the formula (I) or a pharmaceutically acceptable salt thereof, as an active ingredient.
(2) The pharmaceutical composition described in (1), wherein the active ingredient is 2-[(inden-7-yloxy)methyl]morpholine or a pharmaceutically acceptable salt thereof.
(3) The pharmaceutical composition described in (1), wherein the active ingredient is (±)-2-[(inden-7-yloxy)methyl]morpholine or a pharmaceutically acceptable salt thereof.
(4) The pharmaceutical composition described in (1), wherein the active ingredient is (+)-2-[(inden-7-yloxy)methyl]morpholine or a pharmaceutically acceptable salt thereof.
(5) A pharmaceutical composition for treating pain accompanying a disease selected from the group consisting of rheumatoid arthritis, rheumatoid spondylitis, osteoarthritis and spondylosis deformans; and lumbago accompanying spondylosis deformans, comprising (+)-2-[(inden-7-yloxy)methyl]morpholine or a pharmaceutically acceptable salt thereof as an active ingredient.
(6) A pharmaceutical composition for treating chronic pain accompanying a disease selected from the group consisting of rheumatoid arthritis, rheumatoid spondylitis, osteoarthritis and spondylosis deformans; and chronic lumbago accompanying spondylosis deformans, comprsing (+)-2-[(inden-7-yloxy)methyl]morpholine or a pharmaceutically acceptable salt thereof as an active ingredient.

(7) The morpholine derivative represented by the formula (I) includes a compound represented by the following the formula (II), (III) or (IV), but is preferably a compound of the formula (II) or (III), more preferably a compound of the formula (II), and even more preferably, a compound of the formula (II) in which R¹, R² and R³ are hydrogen. 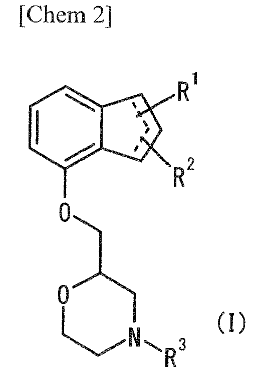
   (wherein R¹, R² and R³ are defined as above) 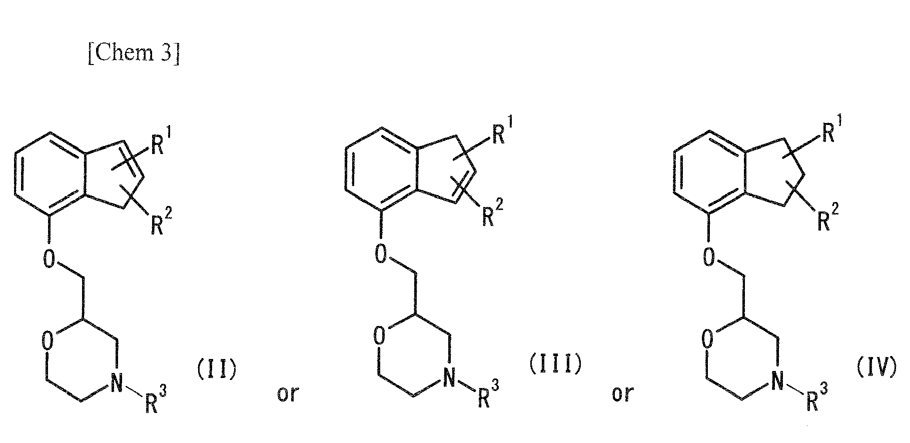 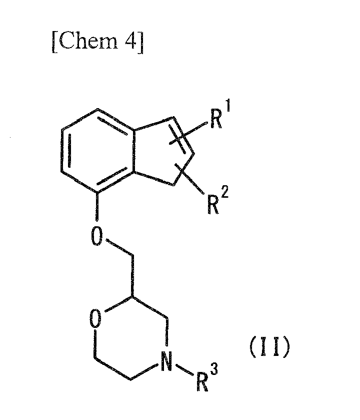 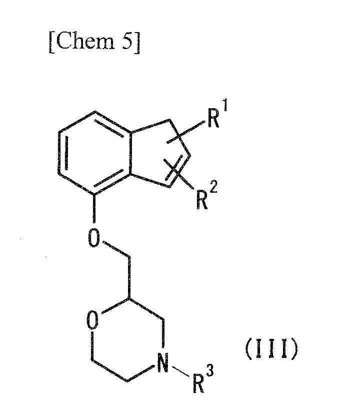
   (wherein R¹, R² and R³ are defined as above).

In the description mentioned above or below in this specification, preferred examples of various definitions falling within the scope of the present invention will be described in detail below.

The term "lower alkyl" refers to a linear or branched aliphatic hydrocarbon having 1 to 6 carbon atoms. Examples thereof include methyl, ethyl, propyl, isopropyl, butyl, isobutyl, tert-butyl, pentyl, and hexyl and the like.

The term "nociceptive pain" refers to pain which is induced by a nociceptive stimulus such as inflammatory chemical mediators released after tissue injury, disease, or inflammation and is detected by sensory receptors (nociceptors) which normally operate in the injured region. Specifically, nociceptive pain includes acute or chronic pain in arthrosis and muscles [for example, pain accompanying rheumatoid arthritis, rheumatoid spondylitis, osteoarthritis, spondylosis deformans, gouty arthritis, juvenile arthritis, scapulohumeral periarthritis or cervical syndrome]; acute or chronic lumbago [for example, pain accompanying spondylosis deformans]; menalgia; pain and tumentia after inflammation, operation or injury [for example, pain after odontectomy]; and cancer pain.

The compound of the formula (I) and/or a pharmaceutically acceptable salt thereof can be obtained by a method disclosed in Patent Citation 1 and Non-patent Citation 3 or a preparation method in accordance therewith.

The compound of the formula (I) may have one or more asymmetric centers, and, in this case, it may be present as an enantiomer or a diastereomer. In the present invention, a mixture of these isomers and the respective isomers separated from each other are all included.

Accordingly, for example, in addition to 2-[(inden-7-yloxy)methyl]morpholine and (±)-2-[(inden-7-yloxy)methyl]morpholine, its enantiomers, (+)-2-[(inden-7-yloxy)methyl]morpholine and (-)-2-[(inden-7-yloxy)methyl]morpholine are included in the compound of the formula (I).

In addition, (±)-2-[(inden-7-yloxy)methyl]morpholine, and its enantiomers, (+)-2-[(inden-7-yloxy)methyl]morpholine, and, (-)-2-[(inden-7-yloxy)methyl]morpholine are included in 2-[(inden-7-yloxy)methyl]morpholine.

The compound of the formula (I) can be formed into salts with various acids by a common procedure. The salt of the compound (1) is a pharmaceutically acceptable salt, and examples thereof include organic acid salts (such as acetates, malonates, tartrates, methanesulfonates, benzenesulfonates, formates, toluenesulfonates, and trifluoroacetates), inorganic acid salts (such as hydrochlorides, hydrobromides, sulfates, and phosphates), and amino acid salts (such as alginates, aspartates, and glutamates). Accordingly, the present invention includes all morpholine derivatives represented by the formula (I) and pharmaceutically acceptable salts thereof.

The compound of the formula (I) can form hydrates and various pharmaceutically acceptable solvates. These hydrates and solvates are also included in the invention.

A pharmaceutical preparation of the present invention can be prepared by a commonly used procedure using a pharmaceutical carrier, excipient, and the like which are commonly used in this field. The administration may be either oral administration by a tablet, a pill, a capsule, a granule, a powder, a liquid, or the like, or parenteral administration by injection (such as intraarticular, intravenous or intramuscular injection), a suppository, an eye drop, an eye ointment, a transdermal liquid, an ointment, a transdermal adhesive patch, a transmucosal liquid, a transmucosal adhesive patch, an inhalant, or the like.

As a solid composition for oral administration in the present invention, a tablet, a powder, a granule, or the like are used. In such a solid composition, one or more active ingredients are mixed with at least one inert diluent such as lactose, mannitol, glucose, hydroxypropyl cellulose, microcrystalline cellulose, starch, polyvinylpyrrolidone, and/or magnesium metasilicate aluminate. The composition may contain an additive other than the inert diluent, for example, a lubricant such as magnesium stearate, a disintegrating agent such as cellulose calcium glycolate, a stabilizing agent, or a solubilizing agent according to a common procedure. The tablet or pill may be coated with a sugar coating such as sucrose, gelatin, hydroxypropyl cellulose, hydroxypropylmethyl cellulose phthalate, or the like, or a film such as a gastric-soluble or enteric-soluble substance, as needed.

A liquid composition for oral administration includes a pharmaceutically acceptable emulsion, solution, suspension, syrup, elixir, and the like, and induces a commonly used inert diluent such as purified water or ethanol. The liquid composition may further contain an auxiliary agent such as a solubilizing agent, a wetting agent, or a suspending agent, a sweetener, a flavor, an aromatic, or a preservative other than the inert diluent.

The injection for parenteral administration contains a sterile aqueous or non-aqueous solution, suspension or emulsion. Examples of the aqueous solution or suspension include distilled water for injection and physiological saline. Examples of the non-aqueous solution or suspension include propylene glycol, polyethylene glycol, vegetable oils such as olive oil, alcohols such as ethanol, and polysorbate 80 (Pharmacopoeia name) and the like. Such a composition may further contain a tonicity agent, a preservative, a wetting agent, an emulsifier, a dispersant, a stabilizing agent, or a solubilizing agent. These are sterilized by, for example, filtration through a bacteria-trapping filter, the addition of a bactericide thereto, or irradiation. Alternatively, a sterile solid composition is prepared, and the resulting composition can be used by being dissolved or suspended in sterile water or a sterile solvent for injection before use.

As a transmucosal preparation such as a transnasal preparation, a solid, a liquid, or a semi-solid preparation are used, and such a preparation can be prepared according to a conventionally known method. For example, a known pH adjusting agent, preservative, thickening agent or excipient are appropriately added and the resulting mixture is formed into a solid, liquid or semi-solid preparation. The transnasal preparation is administered using a common spray apparatus, nasal spray container, tube, intranasal insert, or the like.

A medicinal agent to be used in the invention is administered to a patient with nociceptive pain, and a suitable daily dose is, in the case of usual oral administration, from about 0.001 mg/kg to 100 mg/kg of body weight. The daily dose is administered once per day or two to four times per day by dividing it into two to four portions. In the case of intravenous administration, a suitable daily dose is from about 0.0001 mg/kg to 10 mg/kg of body weight, and it is administered once to several times per day by dividing it into one to several portions. Further, in the case of a transmucosal preparation, a dose of about 0.001 mg/kg to 100 mg/kg of body weight is administered once to several times per day by dividing it into one to several portions. The dose is appropriately determined depending on the individual cases by taking into consideration the symptoms, age, gender, and the like.

### [Examples]

The following Examples are provided only for the purpose of illustrating the present invention and should not be construed as limiting the scope and spirit of the present invention. In addition, thermal analysis was carried out in accordance with the following method.

### Thermal analysis (DSC)

About 3 mg of a sample was added to an aluminum sample pan for single use, and variations in heat quantity between the sample and a reference (vacant aluminum sample pan) were continuously measured and recorded under a nitrogen atmosphere (50 mL/min) in a measurement range of room temperature to 300°C at a temperature elevation rate of 10°C/min. In addition, the handling of apparatuses including data processing was performed in accordance with the method and sequence indicated in respective apparatuses (trade name: Hi-Res DSC 2910, manufactured by TA Instrument).

### Example 1

### Synthesis of (+) -2-[(inden-7-yloxy)methyl]morpholine (hereinafter, referred to as "compound A") hydrobromate

2.92 g of 47% hydrobromic acid was dissolved in a mixed solvent of 500 ml of water - 500 ml of methanol with stirring under ice cooling. Under the same conditions, 1000 ml of diethyl ether and 10 g of the compound A, (-)-(2R, 3R)-di-O-benzoyl tartrate, were added to the solution, followed by vigorously stirring for 30 minutes. The reaction mixture was allowed to stand, and an aqueous layer was separated and was then washed four times with 500 ml of diethyl ether. The resulting aqueous layer was concentrated under reduced pressure at room temperature or below, dissolved in methanol and then filtered. The filtrate was concentrated under reduced pressure at room temperature or below and was azeotropically distilled with toluene at room temperature or below. The resulting oil was crystallized with isopropanol-diethyl ether to obtain a crystal. The resulting crystal was recrystallized from 30 ml of ethanol - 40 ml of diethyl ether, to obtain 3.641 g of the compound A hydrobromate, as a white crystal.
¹H-NMR(DMSO)-d₆): 2.96-3.10 (2H, m), 3.20-3.45 (4H, m), 3.71-3.81 (1H, m), 3.99-4.20 (4H, m), 6.59-6.64 (1H, m), 6.81-6.93 (2H, m), 7.08 (1H, d, J=7.6Hz), 7.24 (1H, t, J=7.6Hz), 8.93 (2H, bs)
MS (FAB+): 232.1
Elemental analysis: Calcd. value (for C14H17NO2.HBr) C 53.86%, H 5.81%, N 4.49%, Br 25.59%; Found value C 53.82%, H 5.87%, N 4.44%, Br 25.59%
Endothermic onset temperature in DSC: 166°C

### Example 2

### Preparation of compound A benzene sulfonate (α-form crystal)

1000 mg of the compound A, (-)-(2R, 3R)-di-O-benzoyl tartrate was added to 20 ml of ethyl acetate and 20 ml of an aqueous saturated sodium hydrogen carbonate solution under ice cooling, followed by rapidly stirring under the same conditions and separation of an organic layer. The resulting organic layer was rapidly washed twice with 20 ml of an ice-cooled aqueous saturated sodium hydrogen carbonate solution, washed with 10 ml of ice-cooled saturated brine, rapidly dried over anhydrous magnesium sulfate and was added to a solution of 269 mg of benzenesulfonic acid in 10 ml of ethanol, with stirring under ice cooling. The resulting solution was concentrated under reduced pressure at room temperature or lower and crystallized with ethanol and diethyl ether, and the crystal thus obtained was washed with diethyl ether. The resulting crystal was recrystallized from acetone (3 ml) - water (0.1 ml) - diethyl ether (3 ml) to obtain 422 mg of the compound A benzenesulfonate as a white crystal (α-form crystal).
¹H-NMR (DMSO-d₆): 2.96-3.10 (2H, m), 3.20-3.44 (4H, m), 3.69-3.79 (1H, m), 3.99-4.09 (2H, m), 4.11-4.19 (2H, m), 6.59-6.64 (1H, m), 6.84 (1H, d, J=8.0Hz), 6.88-6.93 (1H, m), 7.08 (1H, d, J=7.6Hz), 7.20-7.36 (4H, m), 7.56-7.63 (2H, m), 8.85 (2H, bs)
MS (EI): 231.0
Elemental analysis: Calcd. value (for C14H17NO2.C6H6O3S) C 61.68%, H 5.95%, N 3.60%, S 8.23%; Found value C 61.60%, H 5.92%, N 3.47%, S 8.23%
Endothermic onset temperature in DSC: 140°C

### Example 3

### Analgesic action in monoiodoacetate (MIA)-induced osteoarthritis model

The present disease model was produced based on the description of Toxicol Pathol 31 (6), 619-624 (2003). Male SD rats (6-7 week old, obtained by Charles River Laboratories Japan, Inc.) were anesthetized with halothane (trade name manufactured by Takeda Pharmaceutical Co., Ltd.) and monosodium iodoacetate (MIA; manufactured by Sigma, St. Lois) was injected once into cavitas articularis through the subpatellar ligament of the right knee (20 mg/mL). MIA was dissolved in physiological saline and 50 µl of the resulting solution was administered with a needle (26 gauge, 0.5 inch). At three weeks after MIA administration (after onset of osteoarthritis), a diluted solution, which was diluted with a solvent so as to obtain a vehicle and the compound A having a predetermined concentration, was orally administered to rats of respective groups (the number of rats of each group: 8). The solvent herein used was distilled water. One hour after administration, the difference in weight between the left and right hindlimbs was measured using an incapacitance tester (manufactured by Linton Instrumentation, Norfolk, UK). The inhibition rate of the drug to be tested was calculated by setting the difference in weight of a vehicle-administered group to an inhibition rate of 0% and the difference in weight of a normal group to an inhibition rate of 100%. Significant difference testing was carried out by between-group comparison between the vehicle-administered group and drug-administered group using a Student's t-test.

As a result, the compound A hydrobromate- administered group in which the compound A was administered in an amount of 30 mg/kg exhibited a statistically significant improvement effect of 54% and a statistically significant improvement effect on the difference in hindlimb weight, as compared to the vehicle-administered group (significance level: 1%). At 3 weeks after MIA administration, inflammation in the knee joint of rats had been already recovered and, thereafter, pain was derived from subsequent disorders of cartilage or bones. As can be seen from the viewpoint that diclofenac and indomethacin, non-steroidal anti-inflammatory drugs, (NSAIDs) generally used for pain of osteoarthritis are not effective in this stage, the present model is a test system which sufficiently reflects pain of osteoarthritis (J. Vet. Med. Sci., 65, 1195-1199(2003), Neuroscience lett., 370, 236-240(2005)). The results thus obtained demonstrate that the compound A is effective in chronic pain resistant to non-steroidal anti-inflammatory drugs.

### Example 4

### Inhibitory effect on the difference in hindlimb weight of adjuvant-derived arthritis rat

In this test, female Lewis rats (7-8 weeks old) were used. 50 µL of H37 Ra (manufactured by DIFCO Co.), Mycobacterium tuberculosis suspended with liquid paraffin to a concentration of 10 mg/mL was subcutaneously administered around the right hind footpad. On the next day, a diluted solution, which was diluted with a solvent so as to obtain the vehicle, duloxetine and the compound A having a predetermined concentration, was orally administered to rats of respective groups. The number of rats of each group was 8. The solvent herein used was distilled water. When taking into consideration the period for obtaining a maximum in-blood-concentration, at one hour after administration, the difference in weight between the left and right hindlimbs was measured using an incapacitance tester (manufactured by Linton Instrumentation, Norfolk, UK). The inhibition rate of the drug to be tested was calculated by setting the difference in weight of a vehicle-administered group to an inhibition rate of 0% and the difference in weight of a normal group to an inhibition rate of 100%. Significant difference testing was carried out by between-group comparison between the vehicle group and the compound A hydrobromate-administered group, and between the vehicle group and the duloxetine hydrochloride-administered group using a Student's t-test.

As a result, the compound A hydrobromate- administered group in which the compound A was administered in an amount of 10 mg/kg exhibited a statistically significant improvement effect of 65%, as compared to the vehicle-administered group (significance level: 0.1%). Meanwhile, the duloxetine hydrochloride believed to have the same action mechanism (SNRI) as the compound A exhibited an improvement effect of 63%, in the case where the duloxetine was administered in an amount of 30 mg/kg. That is, the compound A exhibited about 3-fold higher improvement effect than the duloxetine. These results ascertained that the compound A hydrobromate exhibits analgesic action on pain of arthritis, such as rheumatoid arthritis, accompanying serious inflammation.

### Example 5

### Analgesic action in bradykinin-induced knee pain model

Bradykinin is an algesic substance which is involved in a variety of pain and plays an important role in generating pain of osteoarthritis (Inflammation Research 57, 351-361 (2008)). In addition, it has been reported that hyaluronan clinically prescribed to pain of osteoarthritis is effective as a joint injection in this model (Ann Rheum Dis, 52, 817-822 (1993)). The induction and evaluation of the present model was basically carried out in accordance with Ann Rheum Dis, 52, 817-822 (1993). A diluted solution, which was diluted with a solvent so as to obtain a vehicle and the compound A having a predetermined concentration, was orally administered to male SD rats (6-7 week old, obtained by Charles River Laboratories Japan, Inc.). This test was carried out for 10 rats of each group. The solvent herein used was distilled water. When taking into consideration the period for obtaining a maximum in-blood-concentration, at one hour after administration for the compound A benzenesulfonate-administered group and at three hours after administration for the duloxetine hydrochloride-administered group, a diluted solution (3 µM, 50 µL) in which bradykinin was diluted with physiological saline was injected into the knee joint of the right hindlimb of rats. The following behavior observed after injection of bradykinin was measured as a pain behavior and scored to evaluate drug efficacy (pain behavior score 0: no lamness to lamness for 10 seconds, 1: lamness for 10 to 30 seconds, 2: lift of the limb within 10 seconds, or lamness for 31 or more seconds, 3: three-legged gait within 10 seconds followed by lamness, 4: three-legged gait for 10 or more seconds followed by lamness). The inhibition rate of the drug to be tested was calculated by setting the difference in weight of a vehicle-administered group to an inhibition rate of 0% and the difference in weight of a normal group to an inhibition rate of 100%. Significant difference testing was carried out by between-group comparison between the vehicle group and the drug group using a Dunnett's t-test after ANOVA testing.

As a result, the compound A benzenesulfonate- administered group in which the compound A was administered in an amount of 10 mg/kg exhibited an improvement effect of 55% and a statistically significant score improvement effect, as compared to the vehicle-administered group (significance level: 1%). Meanwhile, the duloxetine believed to have the same action mechanism (SNRI) as the compound A exhibited improvement effect of 56%, in the case where the duloxetine was administered in an amount of 30 mg/kg. That is, the compound A exhibited about a 3-fold higher improvement effect compared with the duloxetine. These results ascertained that the compound A hydrobromate also exhibits analgesic action on pain induced by bradykinin (such as the pain of osteoarthritis).

### Industrial Applicability

The present invention is useful in providing an excellent pharmaceutical composition for treating nociceptive pain. In addition, the present invention is particularly useful in providing a pharmaceutical composition for treating pain accompanying a disease selected from the group consisting of rheumatoid arthritis, rheumatoid spondylitis, osteoarthritis, spondylosis deformans, gouty arthritis, juvenile arthritis, scapulohumeral periarthritis, and cervical syndrome; lumbago; lumbago accompanying spondylosis deformans; menalgia; pain and tumentia after inflammation, operation or injury; pain after odontectomy; and cancer pain.
In addition, the present invention is particularly useful for alleviating pain in a damaged cartilage region, and is particularly useful for osteoarthritis in which NSAIDs are not effective.

## Claims

1. A pharmaceutical composition for treating nociceptive pain, comprising a compound represented by the formula (I) or a pharmaceutically acceptable salt thereof as an active ingredient: 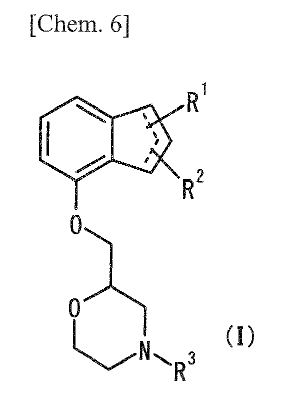
wherein R¹ and R² are the same or different and represent hydrogen, lower alkyl or phenyl;
R³ represents hydrogen, lower alkyl, phenyl or benzyl; and
a dotted line indicates that a double bond may be formed.

2. The pharmaceutical composition according to claim 1, wherein the active ingredient is 2-[(inden-7-yloxy)methyl]morpholine or a pharmaceutically acceptable salt thereof.

3. The pharmaceutical composition according to claim 1, wherein the active ingredient is (±)-2-[(inden-7-yloxy)methyl]morpholine or a pharmaceutically acceptable salt thereof.

4. The pharmaceutical composition according to claim 1, wherein the active ingredient is (+)-2-[(inden-7-yloxy)methyl]morpholine or a pharmaceutically acceptable salt thereof.

5. The pharmaceutical composition according to claim 1, wherein the active ingredient is (+)-2-[(inden-7-yloxy)methyl]morpholine benzenesulfonate.

6. The pharmaceutical composition according to claims 1 to 5, wherein the nociceptive pain is pain accompanying a disease selected from the group consisting of rheumatoid arthritis, rheumatoid spondylitis, osteoarthritis, spondylosis deformans, gouty arthritis, juvenile arthritis, scapulohumeral periarthritis, and cervical syndrome; lumbago; lumbago accompanying spondylosis deformans; menalgia; pain and tumentia after inflammation, operation or injury; pain after odontectomy; or cancer pain.

7. A use of a compound represented by the formula (I) or a pharmaceutically acceptable salt thereof for the manufacture of a medicament for treating nociceptive pain, 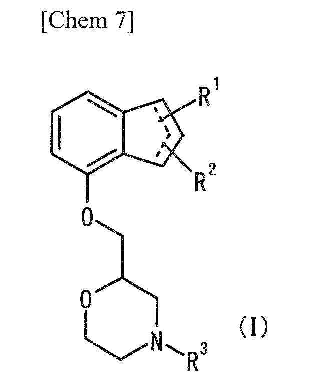
wherein R¹ and R² are the same or different and represent hydrogen, lower alkyl or phenyl;
R³ represents hydrogen, lower alkyl, phenyl or benzyl; and
a dotted line indicates that a double bond may be formed.

8. The use according to claim 7, wherein the compound represented by the formula (I) or a pharmaceutically acceptable salt thereof is 2-[(inden-7-yloxy)methyl]morpholine or a pharmaceutically acceptable salt thereof.

9. The use according to claim 7, wherein the compound represented by the formula (I) or a pharmaceutically acceptable salt thereof is (±)-2-[(inden-7-yloxy)methyl]morpholine or a pharmaceutically acceptable salt thereof.

10. The use according to claim 7, wherein the compound represented by the formula (I) or a pharmaceutically acceptable salt thereof is (+)-2-[(inden-7-yloxy)methyl]morpholine or a pharmaceutically acceptable salt thereof.

11. The use according to claim 7, wherein the compound represented by the formula (I) or a pharmaceutically acceptable salt thereof is (+)-2-[(inden-7-yloxy)methyl]morpholine benzenesulfonate.

12. The use according to claims 7 to 11, wherein the nociceptive pain is pain accompanying a disease selected from the group consisting of rheumatoid arthritis, rheumatoid spondylitis, osteoarthritis, spondylosis deformans, gouty arthritis, juvenile arthritis, scapulohumeral periarthritis and cervical syndrome; lumbago; lumbago accompanying spondylosis deformans; menalgia; pain and tumentia after inflammation, operation or injury; pain after odontectomy; or cancer pain.

13. A method for treating nociceptive pain, comprising administering an effective amount of a compound represented by the formula (I) or a pharmaceutically acceptable salt thereof to human, 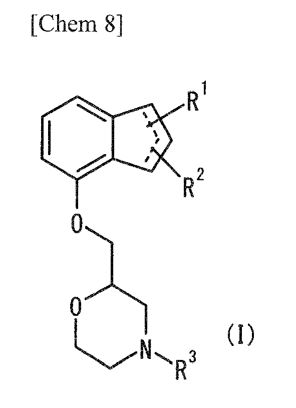
wherein R¹ and R² are the same or different and represent hydrogen, lower alkyl or phenyl;
R³ represents hydrogen, lower alkyl, phenyl or benzyl; and
a dotted line indicates that a double bond may be formed.

14. The method according to claim 13, wherein the compound represented by the formula (I) or a pharmaceutically acceptable salt thereof is 2-[(inden-7-yloxy)methyl]morpholine or a pharmaceutically acceptable salt thereof.

15. The method according to claim 13, wherein the compound represented by the formula (I) or a pharmaceutically acceptable salt thereof is (+)-2-[(inden-7-yloxy)methyl]morpholine or a pharmaceutically acceptable salt thereof.

16. The method according to claim 13, wherein the compound represented by the formula (I) or a pharmaceutically acceptable salt thereof is (+)-2-[(inden-7-yloxy)methyl]morpholine or a pharmaceutically acceptable salt thereof.

17. The method according to claim 13, wherein the compound represented by the formula (I) or a pharmaceutically acceptable salt thereof is (+)-2-[(inden-7-yloxy)methyl]morpholine benzenesulfonate.

18. The method according to claims 13 to 17, wherein the nociceptive pain is pain accompanying a disease selected from the group consisting of rheumatoid arthritis, rheumatoid spondylitis, osteoarthritis, spondylosis deformans, gouty arthritis, juvenile arthritis, scapulohumeral periarthritis and cervical syndrome; lumbago; lumbago accompanying spondylosis deformans; menalgia; pain and tumentia after inflammation, operation or injury; pain after odontectomy; or cancer pain.
